(19) Europäisches Patentamt
European Patent Office
Office européen des brevets

(11) Publication number: **0 354 694 A1**

(12) **EUROPEAN PATENT APPLICATION**

(21) Application number: 89307726.3

(51) Int. Cl.[4]: C07D 495/04 , A61K 31/415

(22) Date of filing: 28.07.89

Claims for the following Contracting States: ES + GR.

The title of the invention has been amended (Guidelines for Examination in the EPO, A-III, 7.3).

(30) Priority: 09.08.88 GB 8818895

(43) Date of publication of application: 14.02.90 Bulletin 90/07

(84) Designated Contracting States: AT BE CH DE ES FR GB GR IT LI LU NL SE

(71) Applicant: THE BOOTS COMPANY PLC
1 Thane Road West
Nottingham NG2 3AA(GB)

(72) Inventor: Bowen, John Gareth
Cherrytrees Barkestone Lane
Plungar Notts(GB)
Inventor: Hockley, Michael Henry
18 Amilda Avenue
Ilkeston Derbyshire(GB)
Inventor: Housley, John Rosindale
146 Wilsthorpe Road
Breaston Derbyshire(GB)
Inventor: Hunneyball, Ian Michael
8 Valley Road
Radcliffe-on-Trent Notts(GB)
Inventor: Titman, Roger Bernard
29 Linwood Crescent
Ravenshead Nottingham(GB)
Inventor: Webber, David George
Moss Close
East Bridgford Nottingham(GB)

(74) Representative: Thacker, Michael Anthony et al
THE BOOTS COMPANY PLC Patents Section
R4 Pennyfoot Street
Nottingham NG2 3AA(GB)

(54) **Therapeutic [1]benzothiopyrano[4,3-c]-pyrazoles.**

(57) This invention relates to compounds of formula I

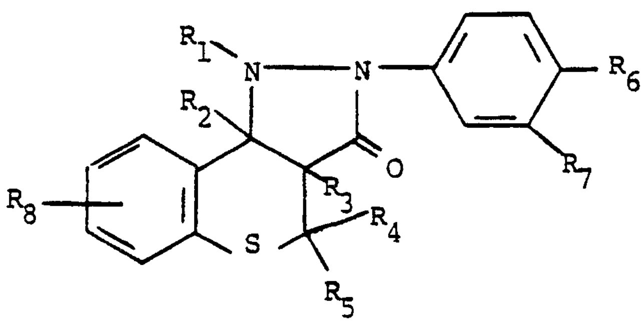

I

wherein $R_1$ represents hydrogen or together with $R_2$ represents a bond; $R_2$ together with either one of $R_1$ and $R_3$ represents a bond; $R_3$ represents methyl or together with either one of $R_2$ and $R_4$ forms a bond; $R_4$ represents hydrogen or together with $R_3$ represents a bond; $R_5$ represents methyl or ethyl when $R_3$ together with either one of $R_2$ and $R_4$ forms a bond or $R_5$ represents hydrogen when $R_3$ represents methyl; $R_6$ represents hydrogen, halo, trifluoromethyl, a $C_{1-6}$ alkylthio group, a $C_{1-6}$ alkyl group or a $C_{1-6}$ alkoxy group; $R_7$ represents hydrogen, halo or trifluoromethyl; and $R_8$ represents hydrogen, halo, trifluoromethyl,

EP 0 354 694 A1

hydroxy or a $C_{1-6}$ alkyl group which have immunomodulatory activity. Compositions containing these compounds and processes to make them are also disclosed.

## THERAPEUTIC AGENTS

The present invention relates to novel therapeutic agents, and in particular to [1]benzothiopyrano[4,3-c]-pyrazoles, to processes for their preparation, to pharmaceutical compositions containing them and to their therapeutic activity as immunomodulatory agents.

It is disclosed in US Patent 4268516 that certain compounds are capable of suppressing the immune response in mammals. These compounds form a small group of 1,2,3,4-tetrahydro[1]benzothiopyrano[4,3-c]pyrazol-3-ones and 2,3-dihydro[1]benzothiopyrano[4,3-c]pyrazol-3-ones of respective structures A and B

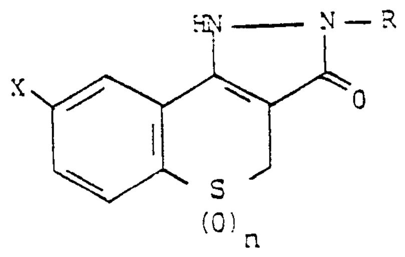

A

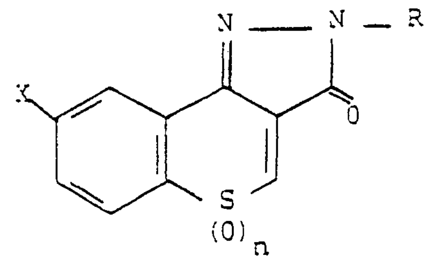

B

wherein n is an integer of value from 0 to 2; R is phenyl; phenyl monosubstituted with chloro, bromo, fluoro, methyl, methoxy, nitro or trifluoromethyl; or phenyl disubstituted with chloro; and X is hydrogen or chloro.

The present invention provides compounds of formula I

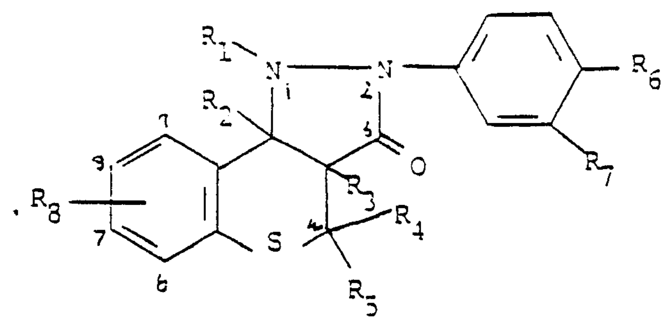

I

wherein $R_1$ represents hydrogen or together with $R_2$ represents a bond; $R_2$ together with either one of $R_1$ and $R_3$ represents a bond; $R_3$ represents methyl or together with either one of $R_2$ and $R_4$ forms a bond; $R_4$ represents hydrogen or together with $R_3$ represents a bond; $R_5$ represents methyl or ethyl when $R_3$ together with either one of $R_2$ and $R_4$ forms a bond or $R_5$ represents hydrogen when $R_3$ represents methyl; $R_6$ represents hydrogen, halo, trifluoromethyl, a $C_{1-6}$ alkylthio group, a $C_{1-6}$ alkyl group or a $C_{1-6}$ alkoxy group; $R_7$ represents hydrogen, halo or trifluoromethyl; and $R_8$ represents hydrogen, halo, trifluoromethyl, hydroxy or a $C_{1-6}$ alkyl group.

It will be understood that a group containing a chain of 3 or more carbon atoms may be straight or branched, for example propyl includes n-propyl and isopropyl, and butyl includes n-butyl, sec-butyl, isobutyl and tert-butyl. The term "halo" covers fluoro, chloro or bromo.

A preferred group of compounds of formula I, in which $R_1$ and $R_2$ represent a bond and $R_3$ and $R_4$ represent a bond, is represented by formula II

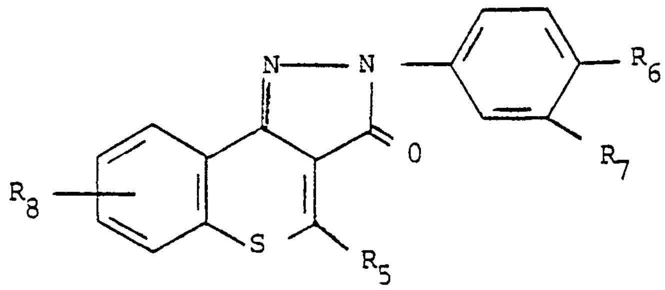

II

wherein $R_5$, $R_6$ $R_7$ and $R_8$ are as hereinbefore defined.

A further preferred group of compounds of formula I, in which $R_1$ represents hydrogen, $R_2$ and $R_3$ represent a bond and $R_4$ represents hydrogen, is represented by compounds of formula III, or tautomers thereof,

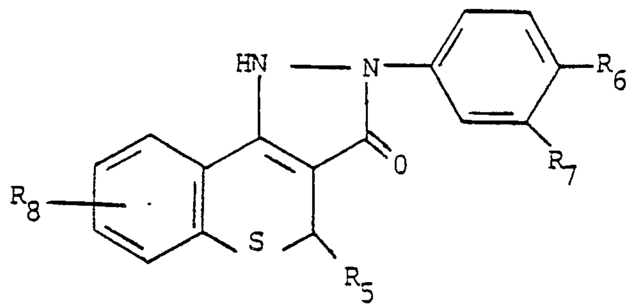

III

wherein $R_5$, $R_6$, $R_7$ and $R_8$ are as hereinbefore defined.

A further preferred group of compounds of formula I, in which $R_1$ and $R_2$ represent a bond, $R_3$ represents methyl and $R_4$ represents hydrogen, is represented by formula IV

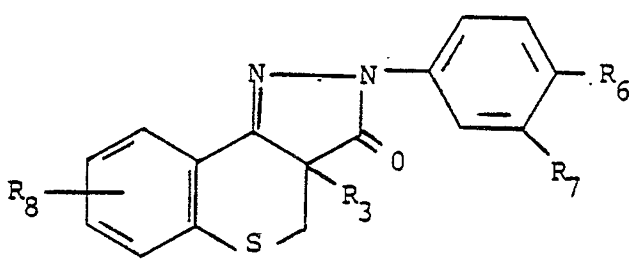

IV

wherein $R_6$, $R_7$ and $R_8$ are as hereinbefore defined.

In compounds of formula I, $R_5$ may represent hydrogen, methyl or ethyl. In preferred compounds of formula I, $R_5$ represents methyl when $R_3$ together with either one of $R_2$ and $R_4$ forms a bond. In further preferred compounds, $R_5$ represents hydrogen when $R_3$ represents methyl.

In compounds of formula I, suitably $R_6$ represents hydrogen, halo, trifluoromethyl, a $C_{1-4}$ alkylthio group, for example methylthio or ethylthio, a $C_{1-4}$ alkyl group, for example methyl or ethyl, or a $C_{1-4}$ alkoxy group, for example methoxy or ethoxy. Preferably $R_6$ represents halo, trifluoromethyl or methylthio, more preferably halo such as chloro or bromo, or trifluoromethyl.

In compounds of formula I, preferably $R_7$ represents hydrogen. In a further preferred group of compounds, $R_7$ represents halo, for example fluoro or chloro, especially chloro. Advantageously when $R_7$ represents halo, $R_6$ represents a substituent other than hydrogen, for example, a $C_{1-6}$ alkyl group such as methyl, or halo.

In compounds of formula I, suitably $R_8$ represents hydrogen, halo, trifluoromethyl, hydroxy or a $C_{1-4}$ alkyl group such as methyl. The substituents may be located in the 6-, 7-, 8- or 9- position of the benz ring, preferably in the 8- or 9- position of the benz ring, and especially in the 8- position. In preferred compounds of formula I, $R_8$ represents hydrogen, fluoro, trifluoromethyl or hydroxy. More preferably $R_8$ represents

hydrogen or fluoro, most preferably hydrogen.

A preferred group of compounds according to the invention are those wherein $R_1$ represents hydrogen or together with $R_2$ represents a bond; $R_2$ together with either one of $R_1$ and $R_3$ represents a bond; $R_3$ represents methyl or together with either one of $R_2$ and $R_4$ forms a bond; $R_4$ represents hydrogen or together with $R_3$ represents a bond; $R_5$ represents methyl or ethyl when $R_3$ together with either one of $R_2$ and $R_4$ forms a bond or $R_5$ represents hydrogen when $R_3$ represents methyl; $R_6$ represents halo, trifluoromethyl or a $C_{1-6}$ alkylthio group, $R_7$ represents hydrogen, halo or trifluoromethyl; and $R_8$ represents hydrogen, fluoro, trifluoromethyl or hydroxy.

In compounds of formula II, the substituents $R_5$, $R_6$, $R_7$ and $R_8$ may be as hereinbefore defined. A more preferred group of compounds of formula II are those wherein $R_5$ represents methyl, $R_6$ represents chloro or trifluoromethyl, and $R_7$ represents hydrogen or chloro and $R_8$ represents hydrogen.

In compounds of formula III, the substituents $R_5$, $R_6$, $R_7$ and $R_8$ may be as hereinbefore defined. A more preferred group of compounds of formula III are those wherein $R_5$ represents methyl, $R_6$ represents bromo or trifluoromethyl and $R_7$ and $R_8$ each represent hydrogen.

In compounds of formula IV, the substituents $R_3$, $R_6$, $R_7$ and $R_8$ may be as hereinbefore defined. A more preferred group of compounds of formula IV are those wherein $R_3$ represents methyl, $R_6$ represents chloro, bromo or trifluoromethyl, $R_7$ represents hydrogen or chloro, especially chloro, and $R_8$ represents hydrogen.

Particular compounds of formula I are :-

2-(4-chlorophenyl)-3a-methyl-3a,4-dihydro[1]benzothiopyrano[4,3-c]pyrazol-3(2H)-one (including R- and S-forms)

2-(4-fluorophenyl)-3a-methyl-3a,4-dihydro[1]benzothiopyrano[4,3-c]pyrazol-3(2H)-one (including R- and S-forms)

2-(3,4-dichlorophenyl)-3a-methyl-3a,4-dihydro[1]benzothiopyrano[4,3-c]pyrazol-3(2H)-one (including R- and S-forms)

2-(3-chlorophenyl)-3a-methyl-3a,4-dihydro[1]benzothiopyrano[4,3-c]pyrazol-3(2H)-one (including R- and S-forms)

8-chloro-2-(3,4-dichlorophenyl)-3a-methyl-3a,4-dihydro[1]benzothiopyrano[4,3-c]pyrazol-3(2H)-one (including R- and S- forms)

3a-methyl-2-(4-trifluoromethylphenyl)-3a,4-dihydro[1]benzothiopyrano[4,3-c]pyrazol-3(2H)-one (including R- and S- forms)

2-(4-bromo-3-chlorophenyl)-3a-methyl-3a,4-dihydro[1]benzothiopyrano[4,3-c]pyrazol-3(2H)-one (including R- and S- forms)

2-(3-chloro-4-fluorophenyl)-3a-methyl-3a,4-dihydro[1]benzothiopyrano[4,3-c]pyrazol-3(2H)-one (including R- and S- forms)

2-(3-chloro-4-methylphenyl)-3a-methyl-3a,4-dihydro[1]-benzothiopyrano[4,3-c]pyrazol-3(2H)-one (including R-and S- forms)

2-(4bromophenyl)-3a-methyl-3a,4-dihydro[1]benzothiopyrano[4,3-c]pyrazol-3(2H)-one (including R- and S-forms)

3a-methyl-2-(4-methylthiophenyl)-3a,4-dihydro[1]benzothiopyrano[4,3-c]pyrazol-3(2H)-one (including R- and S- forms)

2-(4-chlorophenyl)-8-fluoro-3a-methyl-3a,4-dihydro[1]benzothiopyrano[4,3-c]pyrazol-3(2H)-one (including R- and S- forms)

2-(4-chlorophenyl)-4-methyl-1,4-dihydro[1]benzothiopyrano[4,3-c]pyrazol-3(2H)-one (including R- and S-forms)

2-(3,4-dichlorophenyl)-4-methyl-1,4-dihydro[1]benzothiopyrano[4,3-c]pyrazol-3(2H)-one (including R- and S-forms)

2-(4-fluorophenyl)-4-methyl-1,4-dihydro[1]benzothiopyrano[4,3-c]pyrazol-3(2H)-one (including R- and S-forms)

2-(4-chlorophenyl)-4-ethyl-1,4-dihydro[1]benzothiopyrano[4,3-c]pyrazol-3(2H)-one (including R- and S-forms)

4-methyl-2-(4-trifluoromethylphenyl)-1,4-dihydro[1]-benzothiopyrano[4,3-c]pyrazol-3(2H)-one (including R- and S- forms)

2-(4-bromophenyl)-4-methyl-1,4-dihydro[1]benzothiopyrano[4,3-c]pyrazol-3(2H)-one (including R- and S-forms)

2-(4-chlorophenyl)-4-methyl[1]benzothiopyrano[4,3-c]pyrazol-3(2H)-one

2-(4-fluorophenyl)-4-methyl[1]benzothiopyrano[4,3-c]pyrazol-3(2H)-one

2-(4-chlorophenyl)-4-ethyl[1]benzothiopyrano[4,3-c]pyrazol-3(2H)-one

4-methyl-2-(4-trifluoromethylphenyl)[1]benzothiopyrano[4,3-c]pyrazol-3(2H)-one
2-(4-bromophenyl)-4-methyl[1]benzothiopyrano[4,3-c]pyrazol-3(2H)-one
2-(4-methoxyphenyl)-4-methyl[1]benzothiopyrano[4,3-c]pyrazol-3(2H)-one
2-(4-chlorophenyl)-4,8-dimethyl[1]benzothiopyrano[4,3-c]pyrazol-3(2H)-one
2-(3-chlorophenyl)-4-methyl[1]benzothiopyrano[4,3-c]pyrazol-3(2H)-one
2-(3,4-dichlorophenyl)-4-methyl[1]benzothiopyrano[4,3-c]pyrazol-3(2H)-one

Compounds of formula I may contain one or more chiral centres and exist in different optically active forms. When compounds of formula I contain one chiral centre the compounds exist in two enantiomeric forms and the present invention includes both enantiomers and mixtures of enantiomers. The enantiomers may be resolved by methods known to those skilled in the art, for example by high performance liquid chromatography on a chiral support, for example silica with a bound chiral ligand.

When compounds of formula I contain more than one chiral centre, the compounds may exist in diastereoisomeric forms. The present invention includes each diastereoisomer and mixtures of the diastereoisomers. The diastereoisomers may be separated by methods known to those skilled in the art, for example by crystallisation followed by resolution.

The present invention also includes pharmaceutical compositions containing a therapeutically effective amount of a compound of formula I together with a pharmaceutically acceptable diluent or carrier.

In therapeutic use, the active compound may be administered orally, rectally, parenterally or topically, preferably orally or topically. Thus the therapeutic compositions of the present invention take the form of any of the known pharmaceutical compositions for oral, rectal, parenteral or topical administration. Pharmaceutically acceptable carriers suitable for use in such compositions are well known in the art of pharmacy. The compositions of the invention may contain 0.1-90% by weight of active compound. The compositions of the invention are generally prepared in unit dosage form. The excipients used in the preparation of these compositions are the excipients known in the pharmacist's art.

Compositions for oral administration are preferred compositions of the invention and these are known pharmaceutical forms for such administration, for example tablets, capsules, syrups and aqueous or oily suspensions. Tablets may be prepared by mixing the active compound with an inert diluent such as calcium phosphate in the presence of disintegrating agents, for example maize starch, and lubricating agents, for example magnesium stearate, and tableting the mixture by known methods. The tablets may be formulated in a manner known to those skilled in the art so as to give a sustained release of the compounds of the present invention. Such tablets may, if desired, be provided with enteric coatings by known methods, for example by the use of cellulose acetate phthalate. Similarly, capsules, for example hard or soft gelatin capsules, containing the active compound with or without added excipients, may be prepared by conventional means and, if desired, provided with enteric coatings in a known manner. The tablets and capsules may conveniently each contain 0.1 to 500 mg of the active compound. Other compositions for oral administration include, for example, aqueous suspensions containing the active compound in an aqueous medium in the presence of a non-toxic suspending agent such as sodium carboxymethylcellulose, and oily suspensions containing a compound of the present invention in a suitable vegetable oil, for example arachis oil.

Compositions for topical administration are also preferred compositions of the invention. The pharmaceutically active compound may be dispersed in a pharmaceutically acceptable cream, ointment or gel. A suitable cream may be prepared by incorporating the active compound in a topical vehicle such as petrolatum and/or light liquid paraffin, dispersed in an aqueous medium using surfactants. An ointment may be prepared by mixing the active compound with a topical vehicle such as a mineral oil, petrolatum and/or a wax e.g. paraffin wax or beeswax. A gel may be prepared by mixing the active compound with a topical vehicle comprising a gelling agent e.g. basified Carbomer BP, in the presence of water. Topically administrable compositions may also comprise a matrix in which the pharmaceutically active compounds of the present invention are dispersed so that the compounds are held in contact with the skin in order to administer the compounds transdermally. A suitable transdermal composition may be prepared by mixing the pharmaceutically active compound with a topical vehicle, such as described above, together with a potential transdermal accelerant such as dimethyl sulphoxide or propylene glycol.

Compositions of the invention suitable for rectal administration are known pharmaceutical forms for such administration, for example suppositories with cocoa butter or polyethylene glycol bases.

Compositions of the invention suitable for parenteral administration are known pharmaceutical forms for such administration, for example sterile suspensions or sterile solutions in a suitable solvent.

In some formulations it may be beneficial to use the compounds of the present invention in the form of particles of very small size, for example as obtained by fluid energy milling.

In the compositions of the present invention the active compound may, if desired, be associated with

other compatible pharmacologically active ingredients.

The compounds of formula I are immunomodulatory agents, generally manifested as immunosuppressants, but some compounds, in certain disease states, may exhibit immunostimulant activity. The compounds according to the invention are useful in the treatment of diseases resulting from an aberrant immune reaction. The pharmaceutical compositions containing a therapeutically effective amount of a compound of formula I may be used to treat diseases with an immunological association for example tissue rejection, such as kidney rejection; autoimmune diseases, such as rheumatoid arthritis and systemic lupus erythematosus; cutaneous disorders, such as contact sensitivity, eczema and psoriasis; and neoplasia, such as melanoma.

In such treatment the amount of the compound of formula I administered per day will be such as to give a therapeutic effect and is generally in the range 0.1 to 2000 mg, preferably 1 to 500 mg.

Accordingly, in another aspect, the present invention also includes a method of treating diseases with an immunological association, comprising the administration of a therapeutically effective amount of a compound of formula I.

Processes for the preparation of compounds of formula I will now be described. These processes form a further aspect of the present invention.

Compounds of formula I which are represented by formula II may be prepared by oxidising compounds of formula I which are represented by formula III, for example by reaction with chloranil.

Compounds of formula I which are represented by formula III may be prepared by reducing compounds of formula I which are represented by formula II, for example by reaction with sodium borohydride.

Compounds of formula I which are represented by formula III may be prepared by reacting compounds of formula V

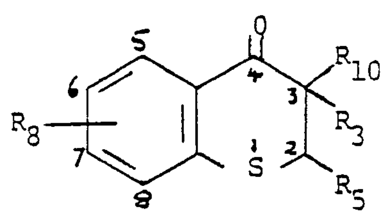

V

in which $R_5$ represents methyl or ethyl, $R_3$ represents hydrogen, $R_{10}$ represents carbamoyl or $COOR_9$, in which $R_9$ represents a $C_{1-4}$ alkyl group or a benzyl group, with a hydrazine of formula VI

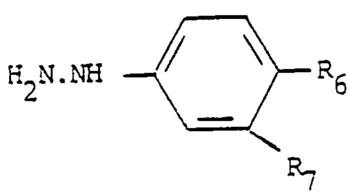

VI

for example by heating at 50-250°C, for example in acetic acid, or in an inert organic liquid containing an acid catalyst, e.g. xylene containing p-toluenesulphonic acid.

Compounds of formula I which are represented by formula IV may be prepared by reacting compounds of formula V, in which $R_5$ represents hydrogen, $R_3$ represents methyl and $R_{10}$ represents carbamoyl or $COOR_9$, in which $R_9$ represents a $C_{1-4}$ alkyl group or a benzyl group, with a hydrazine of formula VI, for example by heating at 50-250°C in an inert organic liquid containing an acid catalyst for example xylene containing p-toluenesulphonic acid.

Compounds of formula V in which $R_{10}$ represents carbamoyl may be prepared from compounds of formula V in which $R_{10}$ represents cyano by methods known to those skilled in the art.

Compounds of formula V in which $R_{10}$ represents $COOR_9$ may be prepared by heating compounds of formula VII

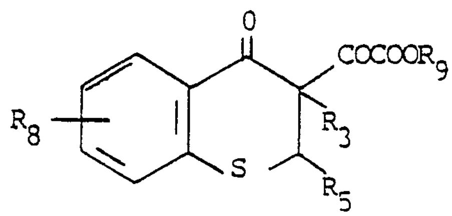

VII

in which $R_9$ represents a $C_{1-4}$ alkyl group or a benzyl group, for example by heating at 100-300 °C with glass powder or glass wool.

Compounds of formula V in which $R_3$ represents hydrogen and $R_{10}$ represents $COOR_9$, in which $R_9$ represents a $C_{1-4}$ alkyl group or a benzyl group, may be prepared by cyclisation of compounds of formula VIII

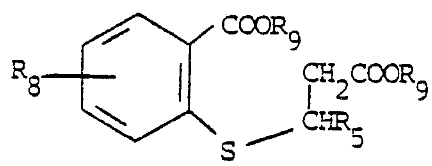

VIII

with a base, for example sodium hydride.

Compounds of formula V in which $R_5$ represents hydrogen, $R_3$ represents methyl and $R_{10}$ represents $COOR_9$, may be prepared by reacting compounds of formula V in which $R_3$ represents hydrogen with a methylating agent, for example methyl iodide in the presence of a base e.g. sodium methoxide.

Compounds of formula V in which $R_5$ represents hydrogen, $R_3$ represents hydrogen and $R_{10}$ represents $COOR_9$, in which $R_9$ represents a $C_{1-4}$ alkyl group or a benzyl group may be prepared by reducing compounds of formula IX

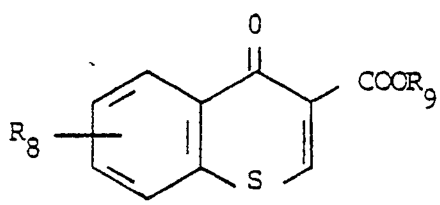

IX

for example by reaction with sodium borohydride.

Compounds of formula V in which $R_5$ represents methyl or ethyl, $R_3$ represents hydrogen and $R_{10}$ represents $COOR_9$, in which $R_9$ represents a $C_{1-4}$ alkyl group or a benzyl group, may be prepared by alkylating a compound of formula IX for example by reaction with lithium dialkyl-copper or methyl magnesium bromide in the presence of cuprous chloride.

Compounds of formula VI may be prepared by methods known to those skilled in the art.

Compounds of formula VII may be prepared by reacting compounds of formula X

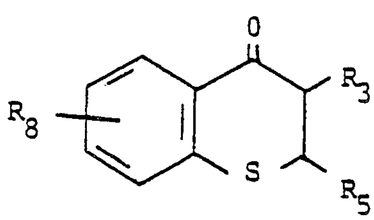

X

with an oxalate ester of formula $(COOR_9)_2$, in which $R_9$ represents a $C_{1-4}$ alkyl group or a benzyl group in the presence of a base, for example a sodium alkoxide of formula $NaOR_9$, for example sodium methoxide.

Compounds of formula VIII may be prepared by reacting compounds of formula XI

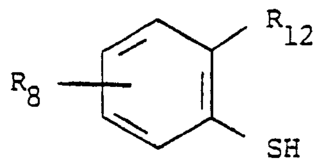

XI

in which $R_{12}$ represents $COOR_9$ in which $R_9$ represents a $C_{1-4}$ alkyl group or a benzyl group, with an acrylic ester of formula

$R_5CH=CHCOOR_9$

for example by heating at 50-250° C in the presence of a basic catalyst for example piperidine

Compounds of formula IX may be prepared by methods known to those skilled in the art.

Compounds of formula X may be prepared by the cyclisation of compounds of formula XII

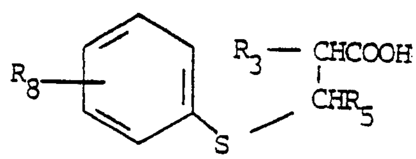

XII

for example by heating at 50-250° C with sulphuric acid or polyphosphoric acid.

Compounds of formula XI may be prepared by methods known to those skilled in the art.

Compounds of formula XII may be prepared by reacting compounds of formula XI in which $R_{12}$ represents hydrogen with an acrylic acid of formula

$R_5CH=C(R_3)COOH$

for example by heating at 100-300° C in the presence of a basic catalyst, for example piperidine.

Certain intermediate compounds of formulae II, III, IV, V, VII, VIII, X and XII are believed to be novel compounds. All novel compounds herein are claimed as a further aspect of the invention.

The invention is illustrated by the following non-limitative Examples.

The therapeutic activity of the compounds of the present invention has been demonstrated by a cutaneous hypersensitivity test (CH test) in which the compounds are administered parenterally to BALB/c mice. This test was carried out in the following way.

Female BALB/c mice, weight range 16-24 g, were used in groups of eight. The abdomen of each mouse was shaved and 20 µl of a solution of a sensitising agent, 5% w/v 4-ethoxymethylene-2-phenyl-2-oxazolin-5-one (oxazolone) in acetone:ethanol (1:1 by volume), was applied to the shaved area. Immediately after sensitisation, the test compound in one of the dosages listed below was injected intraperitoneally as a suspension in 1.5% v/v sorbitan esters, under the trade name Tween 80, in sterile water (100 µl). 100 µl of the same suspension was injected likewise every 24 hours for a further 7 days. The dosages used were selected from the following values: 50, 30, 10, 3, 1, 0.3, 0.1, 0.03 or 0.01 mg/kg.

Two groups of eight BALB/c mice were used as a control simultaneously with each test in a similar manner to that described above except that no test compound was included in the daily injections.

On the seventh day after sensitisation, 10 µl of a solution of 1% w/v oxazolone in acetone: olive oil (either 1:1 or 3:1 by volume) was applied to one ear (the challenged ear) of each of the test mice and the control mice. After 24 hours the thickness of the challenged ear and the thickness of the non-challenged ear of the same animal were measured with an engineer's screw gauge micrometer. The difference in thickness between the challenged ear and the non-challenged ear in each animal is a measure of the response of that animal to oxazolone. A comparison between the response of mice treated with the test compound and mice treated with the control indicates the effectiveness of the test compound as an immunomodulatory agent. The compounds were considered to be active at a particular dose if a 20% or greater reduction in ear swelling, which was statistically significant ($p < 0.05$) according to Dunnett's test, between treated and control groups was obtained in two out of three CH tests, (or, where more than three tests have been carried out, a majority of the tests) at that dose (see for example Int. Arch. Allergy, 38, p246-259 (1970)).

Each of the compounds of formula I illustrated in Table A below was active at 50 mg/kg in two out of three tests at 50 mg/kg unless indicated otherwise (see Notes following the Table). The minimum effective dose (ie the lowest dose of those used at which the compound is active as defined above) for each compound is given in Table A. The Example (Ex) number or numbers listed adjacent to each compound indicates the process or processes illustrating the preparation of that compound in the Examples.

Table A

| Ex | Compound Name | Minimum Effective Dose (mg/kg) |
|---|---|---|
| 11/23 | 2-(4-chlorophenyl)-3a-methyl-3a,4-dihydro[1]benzothiopyrano[4,3-*c*]-pyrazol-3(2*H*)-one | 3 |
| 12 | 2-(4-fluorophenyl)-3a-methyl-3a,4-dihydro[1]benzothiopyrano[4,3-*c*]-pyrazol-3(2*H*)-one | 50 |

Table A (Continued)

| | | |
|---|---|---|
| 13/ 24 | 2-(3,4-dichlorophenyl)-3a-methyl-3a,4-dihydro[1]benzothiopyrano-[4,3-*c*]pyrazol-3(2*H*)-one | 0.3 |
| 14 | 2-(3-chlorophenyl)-3a-methyl-3a,4-dihydro[1]benzothiopyrano[4,3-*c*]-pyrazol-3(2*H*)-one | 3 |
| 15 | 8-chloro-2-(3,4-dichlorophenyl)-3a-methyl-3a,4-dihydro[1]benzothiopyrano[4,3-*c*]pyrazol-3(2*H*)-one | 50 |
| 16 | 3a-methyl-2-(4-trifluoromethylphenyl)-3a,4-dihydro[1]benzothiopyrano-[4,3-*c*]pyrazol-3(2*H*)-one | 0.3 |
| 17 | 2-(4-bromo-3-chlorophenyl)-3a-methyl-3a,4-dihydro[1]benzothiopyrano[4,3-*c*]-pyrazol-3(2*H*)-one | 0.3 |
| 18 | 2-(3-chloro-4-fluorophenyl)-3a-methyl-3a,4-dihydro[1]benzothiopyrano[4,3-*c*]-pyrazol-3(2*H*)-one | 3 |
| 19 | 2-(3-chloro-4-methylphenyl)-3a-methyl-3a,4-dihydro[1]benzothiopyrano[4,3-*c*]-pyrazol-3(2*H*)-one | 3 |
| 20 | 2-(4-bromophenyl)-3a-methyl-3a,4-dihydro[1]benzothiopyrano[4,3-*c*]-pyrazol-3(2*H*)-one | 1 |
| 21 | 3a-methyl-2-(4-methylthiophenyl)-3a,4-dihydro[1]benzothiopyrano[4,3-*c*]-pyrazol-3(2*H*)-one | 50 |

Table A (Continued)

| | | | |
|---|---|---|---|
| 22 | 2-(4-chlorophenyl)-8-fluoro-3a-methyl-3a,4-dihydro[1]benzothiopyrano[4,3-c]-pyrazol-3(2H)-one | 50 | (a) |
| 25 | 2-(4-chlorophenyl)-4-methyl-1,4-dihydro[1]benzothiopyrano[4,3-c]-pyrazol-3(2H)-one | 30 | |
| 26 | 2-(3,4-dichlorophenyl)-4-methyl-1,4-dihydro[1]benzothiopyrano[4,3-c]-pyrazol-3(2H)-one | 50 | (a) |
| 27 | 2-(4-fluorophenyl)-4-methyl-1,4-dihydro[1]benzothiopyrano[4,3-c]-pyrazol-3(2H)-one | 50 | |
| 28 | 2-(4-chlorophenyl)-4-ethyl-1,4-dihydro[1]benzothiopyrano[4,3-c]-pyrazol-3(2H)-one | 50 | |
| 29/34/44 | 4-methyl-2-(4-trifluoromethylphenyl)-1,4-dihydro[1]benzothiopyrano[4,3-c]-pyrazol-3(2H)-one | 3 | |
| 30 | 2-(4-bromophenyl)-4-methyl-1,4-dihydro[1]benzothiopyrano[4,3-c]-pyrazol-3(2H)-one | 3 | |
| 35 | 2-(4-chlorophenyl)-4-methyl[1]-benzothiopyrano[4,3-c]pyrazol-3(2H)-one | 10 | |
| 36 | 2-(4-fluorophenyl)-4-methyl[1]benzothiopyrano[4,3-c]pyrazol-3(2H)-one | 10 | |
| 37 | 2-(4-chlorophenyl)-4-ethyl[1]benzothiopyrano[4,3-c]pyrazol-3(2H)-one | 50 | |

Table A (Continued)

| | | |
|---|---|---|
| 38 | 4-methyl-2-(4-trifluoromethylphenyl)[1]benzothiopyrano[4,3-*c*]-pyrazol-3(2*H*)-one | 0.3 |
| 39 | 2-(4-bromophenyl)-4-methyl[1]benzothiopyrano[4,3-*c*]pyrazol-3(2*H*)-one | 50 |
| 40 | 2-(4-methoxyphenyl)-4-methyl[1]benzothiopyrano[4,3-*c*]pyrazol-3(2*H*)-one | 50 |
| 41 | 2-(4-chlorophenyl)-4,8-dimethyl[1]-benzothiopyrano[4,3-*c*]pyrazol-3(2*H*)-one | 3 |
| 42 | 2-(3-chlorophenyl)-4-methyl[1]benzothiopyrano[4,3-*c*]pyrazol-3(2*H*)-one | 50 |
| 43 | 2-(3,4-dichlorophenyl)-4-methyl[1]-benzothiopyrano[4,3-*c*]pyrazol-3(2*H*)-one | 3 |

Notes:

(a) Active in one out of two tests at 50 mg/kg and active in a single test at 30 mg/kg.

The compounds of the present invention also show activity in a variety of other in-vivo screens, which show the utility of the compounds as immunomodulants, particularly in suppressing the immune response. Administration of the compounds has been carried out orally, parenterally or topically. Some compounds have been found to be active in a test which determines their effects on humoral immunity by assaying the sera collected at the end of the oxazolone induced cutaneous hypersensitivity test described above (CH test) for changes in the amount of anti-oxazolone antibody produced, and a Graft versus Host test similar to that used by Smith S R, Terminelli C, Kipilman C T and Smith Y., J. Immunopharmacology 1981;3(2),133-170.

In the Examples parts and percentages are by weight and compositions of mixed solvents are given by volume. Characterisation was by elemental analysis and one or more of the following spectroscopic techniques: nuclear magnetic resonance, infra-red and mass spectroscopy.

Example 1

a) A stirred mixture of crotonic acid (26.0 g) and thiophenol (33.0 g) was treated with piperidine (5 drops) and then heated with stirring to 235°C during 1 hour. The mixture was maintained at this temperature for 20 minutes and then allowed to cool during 1 hour. After further cooling to 10°C it was treated with concentrated sulphuric acid (160 ml) whilst maintaining the temperature below 55°C. After cooling in an ice bath, the mixture was stirred at ambient temperature for 66 hours. The product was poured on to ice (5 volumes), stirred with dichloromethane and the organic phase was separated. The aqueous phase was extracted with dichloromethane and the combined extracts were washed with water, dried and evaporated to produce an oily residue. The residue was distilled to give 2-methyl-4-thiochromanone as an oil, b.p. 100-110°C (0.2-0.4 mm Hg).

b) A hot solution of dimethyl oxalate (1.37 g) in a solution obtained from sodium (0.27 g) in dry methanol (10 ml) was cooled to ambient temperature and treated dropwise with stirring with a solution of 2-methyl-4-thiochromanone (1.07 g) in dry methanol (6 ml). Stirring was continued for 3 hours and then the product was kept at ambient temperature for 64 hours. The solvent was evaporated off and the residue obtained partitioned between water and toluene. The aqueous layer was basified with 5M sodium hydroxide, separated, washed with toluene and acidified with dilute hydrochloric acid. Extraction with ether gave methyl 2-methyl-4-oxo-3-thiochromanglyoxylate as an oil.

c) A mixture of methyl 2-methyl-4-oxo-3-thiochromanglyoxylate (4.9 g) and glass powder (2.4 g) was heated with stirring at 180-185°C for 30 minutes and then cooled, extracted with acetone and filtered. The filtrate was evaporated and the oily residue distilled to give methyl 2-methyl-4-oxo-3-thiochromancarboxylate, b.p. 170-190°C (0.03 mm Hg), as an oily product.

Example 2

a) In a similar manner to Example 1a, a mixture of crotonic acid (26 g), 4-thiocresol (37 g) and piperidine (5 drops) was heated then cooled and treated with concentrated sulphuric acid (150 ml) to give a yellow solid which was recrystallised from petrol (b.p. 60-80°C) to give 2,6-dimethyl-4-thiochromanone, m.p. 58-60°C.

b) In a similar manner to Example 1b, a solution of 2,6-dimethyl-4-thiochromanone (26 g) in dry methanol (100 ml) was added to a mixture containing dimethyl oxalate (31 g), sodium (6.1 g) and dry methanol (250 ml) and reacted to give a solid product which was recrystallised from ethyl acetate to give methyl 2,6-dimethyl-4-oxo-3-thiochromanglyoxylate, m.p. 95-96°C.

c) In a similar manner to Example 1c, methyl 2,6-dimethyl-4-oxo-3-thiochromanglyoxylate (18.0 g) and glass powder (5.0 g) gave methyl 2,6-dimethyl-4-oxo-3-thiochromancarboxylate, b.p. 126-134°C (0.2 mm Hg).

Example 3

a) In a similar manner to Example 1a, a mixture of trans-2-pentenoic acid (30 g), thiophenol (30.8 ml) and piperidine (5 drops) was heated with stirring at 225-230°C, then treated with concentrated sulphuric acid (290 ml) to give an oil which was distilled to give 2-ethyl-4-thiochromanone, b.p. 127°C (0.8 mm Hg) as an oil.

b) In a similar manner to Example 1b, a solution of 2-ethyl-4-thiochromanone (16.0 g) in dry methanol (80 ml) was added to a mixture containing dimethyl oxalate (18.3 g), sodium (3.62 g) and dry methanol (134 ml) to give methyl 2-ethyl-4-oxo-3-thiochromanglyoxylate as an oil.

c) In a similar manner to Example 1c, heating a mixture of methyl 2-ethyl-4-oxo-3-thiochromanglyoxylate (18.1 g) and glass powder (8.4 g) gave an oil which was distilled to give methyl 2-ethyl-4-oxo-3-thiochromancarboxylate, b.p. 130-134°C (0.05-0.1 mm Hg) as an oil.

Example 4

Sodium borohydride (430 mg) was added in one portion to a stirred solution of ethyl 4-oxo-4H-3-thiochromenecarboxylate (10 g) in methanol (250 ml) at 0°C. The mixture was stirred at 0°C for 30 minutes and the solvent was removed under reduced pressure. The residue was taken up in water and extracted with ether. The combined extracts were dried and evaporated to give an oil which was purified by column chromatography using silica with hexane/ether (4:1) as the mobile phase to give, after evaporation of the

solvent, ethyl 4-oxo-3-thiochromancarboxylate, m.p. 33-34° C.

Example 5

Methyl magnesium bromide (10 ml of 1.5M solution in toluene/tetrahydrofuran, 75:25) was added dropwise over 15 minutes to a stirred mixture of ethyl 4-oxo-4H-3-thiochromenecarboxylate (4 g), in dry tetrahydrofuran (100 ml) containing copper(I) chloride (170 mg) under nitrogen at -78° C. After the addition, the mixture was allowed to warm up to ambient temperature and left at this temperature overnight. The mixture was evaporated under reduced pressure and the residue taken up in 0.5M hydrochloric acid (60 ml) and extracted with ether. The combined ether extracts were washed, dried and evaporated to give a yellow oil which was purified by column chromatography using silica with hexane/ether (4:1) as the mobile phase. Evaporation of the solvent gave ethyl 2-methyl-4-oxo-3-thiochromancarboxylate as an oil.

Example 6

a) A mixture of ethyl 2-mercaptobenzoate (1.0 g), ethyl acrylate (0.6 ml) and pyrrolidine (2 drops) was heated at 150° C for 15 minutes. On cooling, the mixture was diluted with ethyl acetate (15 ml) and washed with water. The organic phase was dried and evaporated to give, as an oil, ethyl 3-(2-ethoxycarbonylphenylthio)propionate.

b) A solution of ethyl 3-(2-ethoxycarbonylphenylthio)propionate (1.0 g) in dry xylene (5 ml) was added to a stirred suspension of sodium hydride (0.14 g; 50% dispersion in mineral oil) in dry xylene (10 ml) under nitrogen. After the addition, the reaction mixture was boiled under reflux for 1 hour. On cooling, the reaction mixture was added to water (50 ml) and this mixture was extracted with toluene. The combined toluene extracts were dried and evaporated under reduced pressure to give, as an oil, ethyl 4-oxo-3-thiochromancarboxylate.

Example 7

a) Dimethyl oxalate (19.5 g) was added to a stirred solution of sodium (3.8 g) in methanol (140 ml) at 20° C. A solution of 6-fluoro-4-thiochromanone (15.0 g) in tetrahydrofuran (130 ml) was added over 10 minutes with stirring and the solution stirred at 20° C for 4 hours. The solvent was removed and the residue was partitioned between water (300 ml) and toluene (100 ml). The aqueous layer was basified with 2M sodium hydroxide solution, separated and acidified with concentrated hydrochloric acid. The precipitated solid was collected by filtration and dried to give methyl 6-fluoro-4-oxo-3-thiochromanglyoxylate, m.p. 107-108° C.

b) In a similar manner to Example 1(c) methyl 6-fluoro-4-oxo-3-thiochromanglyoxylate (10 g) and glass powder (2.5 g) were heated at 180° C for 25 minutes to give an oil which was purified by flash chromatography on silica using dichloromethane as the mobile phase to give an oil. This oil was triturated with petroleum ether (b.p. 40-60° C) to give a solid which was collected by filtration to give methyl 6-fluoro-4-oxo-3-thiochromancarboxylate, m.p. 62-69° C.

c) A solution of methyl 6-fluoro-4-oxo-3-thiochromancarboxylate (3.5 g) in dry toluene (20 ml) was added to a solution obtained from sodium (1.38 g) in dry methanol (60 ml). This solution was heated under reflux for 10 minutes, then allowed to cool to ambient temperature and methyl iodide (3.74 ml) added. The mixture was stirred for 21 hours and then more methyl iodide (3.74 ml) added. This mixture was heated under reflux for 2 hours then cooled to ambient temperature, neutralised with glacial acetic acid and evaporated to dryness. The residue was partitioned between water and toluene and the aqueous layer separated and washed with toluene. The combined organic layers were washed with aqueous sodium bicarbonate and water then dried, filtered and evaporated to give an oil which was purified by flash chromatography on silica, using petroleum ether (b.p. 60-80° C) / ethyl acetate (85:15), as the mobile phase, to give a yellow solid which was triturated with petroleum ether (b.p. 60-80° C) and filtered to give methyl 6-fluoro-3-methyl-4-oxo-3-thiochromancarboxylate, m.p. 94° C.

Example 8

A solution of methyl 6-chloro-4-oxo-3-thiochromancarboxylate (5.2 g) in dry toluene (16 ml) and methanol (16 ml) was added to a solution obtained from sodium (1.82 g) in dry methanol (26 ml) and the mixture heated under reflux for 10 minutes. After cooling to ambient temperature, methyl iodide (5.2 ml) was added and the reaction mixture stirred at ambient temperature for 2 hours then left standing at ambient temperature for 4.5 days. The mixture was acidified with glacial acetic acid and then evaporated to dryness. The solid residue was partitioned between toluene and water. The aqueous layer was separated and washed with toluene. The combined organic extracts were washed with saturated sodium bicarbonate solution, then water, then dried and evaporated to give an oil which was triturated with petroleum ether (b.p. 60-80 °C) and filtered to give methyl 6-chloro-3-methyl-4-oxo-3-thiochromancarboxylate, m.p. 85-88 °C.

Example 9

A solution of ethyl 4-oxo-3-thiochromancarboxylate (1.5 g) in methanol (5 ml) was added to a solution of sodium methoxide (1.5 g) in methanol (25 ml) with stirring. After stirring the mixture at room temperature for 10 minutes, methyl iodide (4.6 g) was added. The mixture was stirred at ambient temperature for 1.5 hours and then boiled under reflux for 20 minutes. After standing at ambient temperature for 16 hours, glacial acetic acid (1 ml) was added and the solvent evaporated off under reduced pressure. The residue was added to water and the mixture extracted with ether. The combined ether extracts were washed with saturated sodium bicarbonate solution, then water, then dried and evaporated under reduced pressure to give, after recrystallisation from methanol, methyl 3-methyl-4-oxothiochroman-3-carboxylate, m.p. 79-81 °C.

Example 10

In a similar manner to Example 9, a solution of ethyl 4-oxo-3-thiochromancarboxylate (4.5 g) in dry ethanol (10 ml) was added to a solution of sodium (1.75 g) in dry ethanol (60 ml) with stirring and treated with methyl iodide (13.5 g) to give an oil which was separated by column chromatography on silica using hexane/ether (4:1) as the mobile phase to give, after evaporation of the solvent, ethyl 3-methyl-4-oxo-3-thiochromancarboxylate as an oil.

Example 11

p-Toluenesulphonic acid (0.2 g) was added to a mixture of methyl 3-methyl-4-oxo-3-thiochromancarboxylate (8.0 g) and 4-chlorophenylhydrazine (5.1 g) in dry xylene (800 ml) and the mixture was stirred and heated under reflux for 24 hours with removal of the water formed in the reaction. During the reaction further portions of p-toluenesulphonic acid (0.2 g) and 4-chlorophenylhydrazine (2.5 g) were added after 16 hours and additional portions of p-toluenesulphonic acid (0.2 g) and 4-chlorophenylhydrazine (1.2 g) were added after 21 hours. The mixture was cooled, filtered and then evaporated to give a dark oil. The oil was dissolved in methanol, boiled with charcoal and hot filtered. The filtrate was cooled and kept for 16 hours at 0 °C. The solid obtained was filtered off, washed and dried to give 2-(4-chlorophenyl)-3a-methyl-3a,4-dihydro[1]benzothiopyrano[4,3-c)]pyrazol-3(2H)-one, m.p. 120-121 °C.

Examples 12-22

In a similar manner to Example 11 compounds of formula IV were prepared by reaction of compounds of formula V with compounds of formula VI as summarized in Table 1 below. In formula V $R_3 = CH_3$ ; $R_5 = H$ ; $R_{10} = COOCH_3$ and $R_8$ is as shown in Table 1. In formula VI, $R_6$ and $R_7$ are as shown in Table 1. Where there are two lines in an Example the reactants on the top line were heated for the time stated on that line and then further reactants were added and the mixture heated for the time stated in the second line.

Notes for Table 1:

(1): A solution of the crude product in ethyl acetate was washed with water, dried and evaporated prior to crystallisation.

(2): The product was purified by preparative thin layer chromatography followed by methanol crystallisation.

(3): This weight of the appropriate hydrazine hydrochloride salt was converted into the free base and used directly.

(4): The product was purified by flash chromatography on silica using dichloromethane / methanol (99:1) as the mobile phase. The product was then crystallised from methanol.

(5): As in note (4) but the product was crystallised from ethanol.

TABLE 1

| Ex | VI | | V | Amount of Reactants | | | Reflux time (hours) | m.p. of IV (°C) | Notes |
|---|---|---|---|---|---|---|---|---|---|
| | $R_6$ | $R_7$ | $R_8$ | V (g) | VI (g) | Xylene (ml) | | | |
| 12 | F | H | H | 3.4 | 1.81 | 300 | 16 | | |
| | | | | | 0.90 | | 24 | 127-128 | |
| 13 | Cl | Cl | H | 1.6 | 1.2 | 140 | 24 | 101.5-102.5 | (1)(2) |
| 14 | H | Cl | H | 1.6 | 1.16 | 100 | 48 | 102-104 | |
| 15 | Cl | Cl | 6-Cl | 3.0 | 2.10 | 250 | 16 | | |
| | | | | | 1.05 | | 16 | 130-133 | (1)(2) |
| 16 | $CF_3$ | H | H | 3.0 | 2.23 | 130 | 16 | | |
| | | | | | 1.12 | | 16 | 110-111 | |
| 17 | Br | Cl | H | 2.6 | 2.44 | 113 | 16 | | |
| | | | | | 2.44 | | 16 | 117-120 | (1) |
| 18 | F | Cl | H | 3.0 | 2.04 | 130 | 16 | | |
| | | | | | 1.02 | 130 | 24 | 125-127 | |
| 19 | $CH_3$ | Cl | H | 2.6 | 1.72 | 130 | 18 | | |
| | | | | | 0.90 | | 24 | 111-113 | |

TABLE 1 Continued

| Ex | VI | | V | Amount of Reactants | | | Reflux time (hours) | m.p. of of IV (°C) | Notes |
|---|---|---|---|---|---|---|---|---|---|
| | $R_6$ | $R_7$ | $R_8$ | V (g) | VI (g) | Xylene (ml) | | | |
| 20 | Br | H | H | 3.0 | 2.38 | 130 | 17 | | (1) |
| | | | | | 1.2 | | 24 | 117-119 | |
| 21 | $SCH_3$ | H | H | 2.38 | 2.86(3) | 140 | 16 | 121-122 | (4) |
| 22 | Cl | H | 6-F | 1.5 | 1.1(3) | 50 | 24 | | |
| | | | | | 1.1(3) | 50 | 24 | 121-122 | (5) |

Ex = Example m.p. = melting point

Example 23

A mixture of ethyl 3-methyl-4-oxo-3-thiochromancarboxylate (0.5 g), 4-chlorophenylhydrazine (0.28 g) and glacial acetic acid (0.2 ml) was heated under reflux in xylene (10 ml) for 20 hours, with removal of the water formed in the reaction. After cooling to ambient temperature, the mixture was evaporated under reduced pressure to give an oil which was crystallised from methanol to give 2-(4-chlorophenyl)-3a-methyl-3a,4-dihydro[1]benzothiopyrano[4,3-c]pyrazol-3(2H)-one, m.p. 119-120 ° C.

Example 24

a) A solution of 3-methyl-4-oxo-3-thiochromancarbonitrile (1.0 g) in dry methanol (50 ml) was stirred at -5 to 0 ° C and saturated with hydrogen chloride. After standing at ambient temperature for 5 days the methanol was removed under reduced pressure. The residue was diluted with water and extracted with ethyl acetate. The combined organic extracts were washed with water, dried and evaporated to give a solid which was recrystallised from industrial methylated spirit to give 3-methyl-4-oxo-3-thiochromancarboxamide, m.p. 137-140 ° C.

b) A mixture of 3-methyl-4-oxo-3-thiochromancarboxamide (111 mg), 3,4-dichlorophenylhydrazine (88.5 mg), xylene (10 ml) and p-toluenesulphonic acid (0.2 g) was stirred and heated under reflux for 24 hours with removal of the water formed. The reaction mixture was evaporated under reduced pressure and the residue digested into boiling methanol and hot filtered to remove an insoluble gum. The filtrate was concentrated and cooled to give a solid which was collected by filtration and dried to give 2-(3,4-dichlorophenyl)-3a-methyl-3a,4-dihydro[1]benzothiopyrano[4,3-c]pyrazol-3(2H)-one, m.p. 100-103 ° C.

Example 25

A mixture of methyl 2-methyl-4-oxo-3-thiochromancarboxylate (2.8 g) (Example 1) and 4-chlorophenylhydrazine (1.8 g) in glacial acetic acid (5 ml) was stirred at 115-120 ° C under nitrogen for 25 minutes. The mixture was cooled to ambient temperature, filtered and the solid product was washed with acetic acid and ether to give 2-(4-chlorophenyl)-4-methyl-1,4-dihydro[1]benzothiopyrano[4,3-c]pyrazol-3(2H)-one, m.p. 201-204 ° C.

Examples 26-33

In a similar manner to Example 25 compounds of formula III were prepared by reacting compounds of formula V with compounds of formula VI as summarized in Table 2 below. In compounds of formula V $R_{10}$ = $COOCH_3$, $R_3$ = H and $R_5$ and $R_8$ are as shown in Table 2. In compounds of formula VI $R_6$ and $R_7$ are as shown in Table 2.

NOTES FOR TABLE 2

(1): Reactants heated at 125-130 ° C.
(2): Reactants heated at 115-120 ° C.
(3): The cooled reaction mixture was diluted with ether.
(4): Recrystallisation from methanol.
(5): Recrystallisation from dichloromethane/methanol.
(6): The product was recrystallised from acetonitrile and then purified by flash chromatography on silica using dichloromethane/methanol (9:1) as the mobile phase. The product was then recrystallised from ethyl acetate.

TABLE 2

| Ex | V: $R_5$ | V: $R_8$ | VI: $R_6$ | VI: $R_7$ | Amount of Reactants: V (g) | Amount of Reactants: VI (g) | Amount of Reactants: Glacial acetic acid (ml) | Time (mins) | m.p. of III (°C) | Notes |
|---|---|---|---|---|---|---|---|---|---|---|
| 26 | $CH_3$ | H | Cl | Cl | 1.44 | 1.08 | 3 | 30 | 205-207 | (1) (3) (4) |
| 27 | $CH_3$ | H | F | H | 3.54 | 1.90 | 7 | 30 | 206-208 | (1) (3) |
| 28 | $C_2H_5$ | H | Cl | H | 6.90 | 4.15 | 12 | 30 | 194-196 | (2) (3) (5) |
| 29 | $CH_3$ | H | $CF_3$ | H | 4.00 | 2.98 | 7 | 40 | 200-201 | (1) (3) |
| 30 | $CH_3$ | H | Br | H | 6.00 | 4.75 | 10.5 | 40 | 210-211 | (1) (3) |
| 31 | $CH_3$ | 6-$CH_3$ | Cl | H | 12.5 | 7.8 | 20 | 240 | 190-192 | (2) (6) |
| 32 | $CH_3$ | H | H | Cl | 5.0 | 3.14 | 8 | 90 | 208-212 | (2) |
| 33 | $CH_3$ | H | $OCH_3$ | H | 5.10 | 3.00 | 9 | 30 | 189-191 | (1) (3) |

Ex = Example    m.p. = melting point

## Example 34

A mixture of ethyl 2-methyl-4-oxo-3-thiochroman carboxylate (0.43 g), 4-trifluoromethylphenylhydrazine (0.30 g) and glacial acetic acid (1 ml) was stirred and heated at 125-130 °C for 30 minutes. The mixture was cooled to ambient temperature and ether (15 ml) added. The precipitated solid was collected by filtration,

washed with ether and dried to give 4-methyl-2-(4-trifluoromethylphenyl)-1,4-dihydro[1]benzothiopyrano[4,3-c]pyrazol-3(2H)-one, m.p. 200-202 °C.

Example 35

p-Chloranil (0.5 g) was added to a stirred solution of 2-(4-chlorophenyl)-4-methyl-1,4-dihydro[1]-benzothiopyrano[4,3-c]pyrazol-3(2H)-one (0.65 g), prepared as in Example 25, in dimethyl sulphoxide (7 ml) and stirring was continued at ambient temperature for 1 hour. The solid product was collected and washed with dimethyl sulphoxide, acetone and petroleum ether (b.p. 62-68 °). The crystals obtained were dried to give 2-(4-chlorophenyl)-4-methyl[1]benzothiopyrano[4,3-c]pyrazol-3(2H)-one, m.p. 206-210 °C (decomposes).

Examples 36-43

In a similar manner to Example 35 compounds of formula II were prepared from compounds of formula III as shown in Table 3 below. $R_5$, $R_6$, $R_7$ and $R_8$ are as shown in Table 3.

NOTES FOR TABLE 3

(1): Product washed with ether.

(2): Reaction mixture diluted with acetone. The product was collected by filtration, washed with acetone and then recrystallised from dichloromethane/industrial methylated spirit (4:1).

TABLE 3

| Example | III | | | | Amount of Reactants | | | Stirring time (hours) | m.p. of II (°C) | Notes |
|---|---|---|---|---|---|---|---|---|---|---|
| | $R_5$ | $R_6$ | $R_7$ | $R_8$ | III (g) | DMSO (ml) | p-chloranil (g) | | | |
| 36(27) | $CH_3$ | F | H | H | 1.09 | 12 | 0.9 | 1 | 192-194 | |
| 37(28) | $C_2H_5$ | Cl | H | H | 1.2 | 12 | 0.9 | 1 | 153-155 | |
| 38(29) | $CH_3$ | $CF_3$ | H | H | 1.00 | 11 | 0.688 | 16 | 204-205 | |
| 39(30) | $CH_3$ | Br | H | H | 1.5 | 15 | 0.989 | 16 | 172-174 | |
| 40(33) | $CH_3$ | $OCH_3$ | H | H | 2.0 | 20 | 1.5 | 6 | 174-176 | |
| 41(31) | $CH_3$ | Cl | H | 8-$CH_3$ | 1.36 | 10 | 0.91 | 0.25 | 192-194 | (1) |
| 42(32) | $CH_3$ | H | Cl | H | 1.5 | 30 | 1.06 | 3 | 178-180 | |
| 43(26) | $CH_3$ | Cl | Cl | H | 2.0 | 20 | 1.27 | 2.25 | 229-231 | (2) |

m.p. = melting point

Example 44

Sodium borohydride (0.11 g) was added in portions to a stirred mixture of 4-methyl-2-(4-trifluoromethylphenyl)[1]benzothiopyrano[4,3-c]pyrazol-3(2H)-one (0.36 g) in absolute ethanol (8 ml). The mixture was stirred at ambient temperature for 1 hour and then poured on to water (80 ml). The pH was adjusted to 4-5

with glacial acetic acid. The solid formed was collected by filtration and dried to give 4-methyl-2-(4-trifluoromethylphenyl)-1,4-dihydro[1]benzothiopyrano[4,3-c]pyrazol-3(2H)-one, m.p. 200-201 °C.

Example 45

In the preparation of tablets comprising 100 parts by weight of active compound, 250 parts by weight lactose, 22 parts by weight maize starch, 10 parts by weight polyvinylpyrrolidone and 3 parts by weight magnesium stearate, the active compound, the lactose and some of the starch are de-aggregated, blended and the resulting mixture is granulated with a solution of polyvinylpyrrolidone in ethanol. The dry granulate is blended with the magnesium stearate and the rest of the starch. The mixture is then compressed in a tableting machine to give tablets containing 100mg active compound.

## Claims

1. A compound of formula I

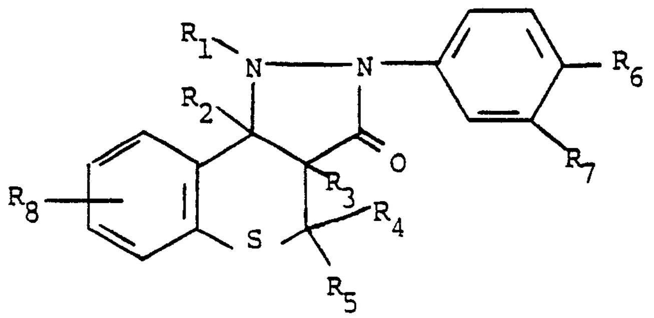

I

wherein $R_1$ represents hydrogen or together with $R_2$ represents a bond; $R_2$ together with either one of $R_1$ and $R_3$ represents a bond; $R_3$ represents methyl or together with either one of $R_2$ and $R_4$ forms a bond; $R_4$ represents hydrogen or together with $R_3$ represents a bond; $R_5$ represents methyl or ethyl when $R_3$ together with either one of $R_2$ and $R_4$ forms a bond or $R_5$ represents hydrogen when $R_3$ represents methyl; $R_6$ represents hydrogen, halo, trifluoromethyl, a $C_{1-6}$ alkylthio group, a $C_{1-6}$ alkyl group or a $C_{1-6}$ alkoxy group; $R_7$ represents hydrogen, halo or trifluoromethyl; and $R_8$ represents hydrogen, halo, trifluoromethyl, hydroxy or a $C_{1-6}$ alkyl group.

2. A compound according to claim 1 wherein $R_6$ represents halo, trifluoromethyl or a $C_{1-6}$ alkylthio group and $R_8$ represents hydrogen, fluoro, trifluoromethyl or hydroxy.

3. A compound according to either one of claims 1 and 2 wherein $R_5$ represents methyl when $R_3$ together with either one of $R_2$ and $R_4$ forms a bond.

4. A compound according to any one of claims 1-3 wherein $R_6$ represents halo or trifluoromethyl.

5. A compound according to any one of the preceding claims wherein $R_7$ represents hydrogen or chloro.

6. A compound according to any one of the preceding claims wherein $R_8$ represents hydrogen.

7. A compound according to any one of the preceding claims wherein $R_1$ and $R_2$ form a bond, $R_3$ and $R_4$ form a bond, $R_5$ represents methyl, $R_6$ represents chloro or trifluoromethyl, $R_7$ represents hydrogen or chloro and $R_8$ represents hydrogen.

8. A compound according to any one of claims 1 to 6 wherein $R_1$ represents hydrogen, $R_2$ and $R_3$ form a bond, $R_4$ represents hydrogen, $R_5$ represents methyl, $R_6$ represents bromo or trifluoromethyl and $R_7$ and $R_8$ each represent hydrogen.

9. A compound according to any one of the preceding claims wherein $R_3$ represents methyl, $R_5$ represents hydrogen, $R_6$ represents chloro, bromo or trifluoromethyl, $R_7$ represents hydrogen or chloro and $R_8$ represents hydrogen.

10. A compound selected from:-
2-(3,4-dichlorophenyl)-3a-methyl-3a,4-dihydro[1]-benzothiopyrano[4,3-c]pyrazol-3(2H)-one,
4-methyl-2-(4-trifluoromethylphenyl)[1]benzothiopyrano[4,3-c]pyrazol-3(2H)-one,
2-(4-bromo-3-chlorophenyl)-3a-methyl-3a,4-dihydro[1]benzothiopyrano[4,3-c]pyrazol-3(2H)-one.

11. A pharmaceutical composition comprising a compound of formula I as claimed in any one of claims 1 to 10 together with a pharmaceutical carrier.

12. A pharmaceutical composition according to claim 11 in unit dosage form.

13. A method of treating diseases with an immunological association comprising the administration of a therapeutically effective amount of a compound of formula I as claimed in any one of claims 1 to 10.

14. A compound of formula I as claimed in any one of claims 1 to 10 for use as an immunomodulatory agent.

15. A compound of formula I as claimed in any one of claims 1 to 10 in the manufacture of a medicament for use in the treatment of diseases with an immunological assocation.

16. A process to prepare compounds of formula I wherein $R_1$ and $R_2$ represent a bond, $R_3$ and $R_4$ represent a bond and $R_5$, $R_6$, $R_7$ and $R_8$ are as defined in claim 1 comprising oxidising compounds of formula I wherein $R_1$ represents hydrogen, $R_2$ and $R_3$ represent a bond, $R_4$ represents hydrogen and $R_5$, $R_6$, $R_7$ and $R_8$ are as herein defined.

17. A process to prepare compounds of formula I wherein $R_1$ represents hydrogen, $R_2$ and $R_3$ represent a bond, $R_4$ represents hydrogen and $R_5$, $R_6$, $R_7$ and $R_8$ are as defined in claim 1 comprising reducing compounds of formula I wherein $R_1$ and $R_2$ represent a bond, $R_3$ and $R_4$ represent a bond and $R_5$, $R_6$, $R_7$ and $R_8$ are as herein defined.

18. A process to prepare compounds of formula I wherein $R_1$ represents hydrogen, $R_2$ and $R_3$ represent a bond and $R_4$ represents hydrogen and $R_5$, $R_6$, $R_7$ and $R_8$ are as defined in claim 1 comprising reacting compounds of formula V

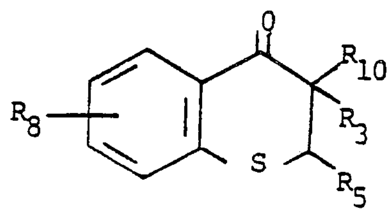

V

in which $R_5$ represents methyl or ethyl, $R_3$ represents hydrogen; $R_{10}$ represents carbamoyl or $COOR_9$ in which $R_9$ represents a $C_{1-4}$ alkyl group or a benzyl group, with a hydrazine of formula VI

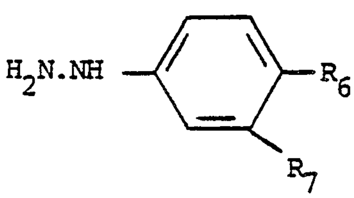

VI

19. A process to prepare compounds of formula I wherein $R_1$ and $R_2$ represent a bond, $R_3$ represents methyl, $R_4$ and $R_5$ represent hydrogen and $R_6$, $R_7$ and $R_8$ are as defined in claim 1 comprising reacting compounds of formula V

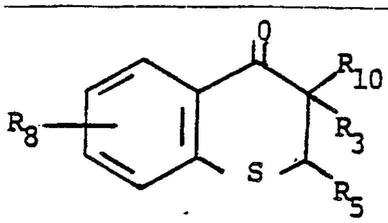

V

wherein $R_5$ represents hydrogen, $R_3$ represents methyl and $R_{10}$ represents carbamoyl or $COOR_9$, in which $R_9$ represents a $C_{1-4}$ alkyl group or a benzyl group, with a hydrazine of formula VI

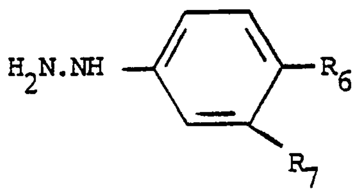

VI

Claims for the following Contracting States: ES, GR

1. A process to prepare compounds of formula I

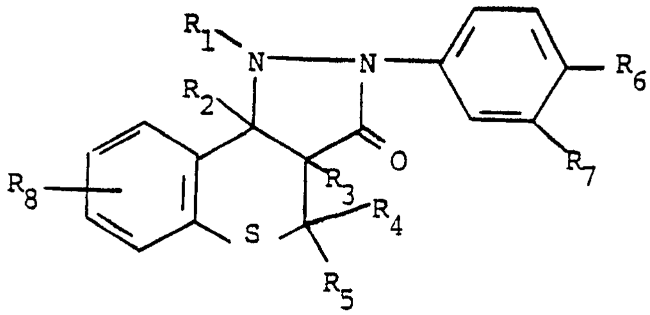

I

wherein $R_1$ and $R_2$ represent a bond, $R_3$ and $R_4$ represent a bond, $R_5$ represents methyl or ethyl; $R_6$ represents hydrogen, halo, trifluoromethyl, a $C_{1-5}$ alkylthio group, a $C_{1-6}$ alkyl group or a $C_{1-6}$ alkoxy group; R7 represent hydrogen, halo or trifluoromethyl; and $R_8$ represents hydrogen, halo, trifluoromethyl, hydroxy or a $C_{1-5}$ alkyl group comprising oxidising compounds of formula I wherein $R_1$ represents hydrogen, $R_2$ and $R_3$ represent a bond, $R_4$ represents hydrogen and $R_5$, $R_6$, $R_7$ and $R_8$ are as herein defined.

2. A process according to claim 1 to prepare a compound wherein $R_5$ represents methyl, $R_6$ represents chloro or trifluoromethyl, $R_7$ represents hydrogen or chloro and $R_8$ represents hydrogen.

3. A process to prepare compounds of formula I as claimed in claim 1 wherein $R_1$ represents hydrogen, $R_2$ and $R_3$ represent a bond, $R_4$ represents hydrogen, $R_5$ represents methyl or ethyl; $R_6$ represents hydrogen, halo, trifluoromethyl, a $C_{1-5}$ alkylthio group, a $C_{1-6}$ alkyl group or a $C_{1-6}$ alkoxy group; R7 represents hydrogen, halo or trifluoromethyl; and $R_8$ represents hydrogen, halo, trifluoromethyl, hydroxy or a $C_{1-6}$ alkyl group,

a) comprising reducing compounds of formula I wherein $R_1$ and $R_2$ represent a bond, $R_3$ and $R_4$ represent a bond and $R_5$, $R_6$, $R_7$ and $R_8$ are as herein defined; or

b) comprising reacting compounds of formula V

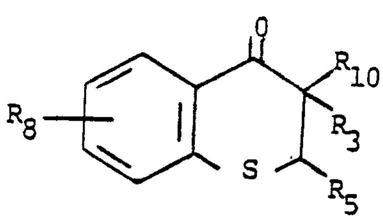

V

in which $R_5$ represents methyl or ethyl, $R_3$ represents hydrogen; $R_{10}$ represents carbamoyl or $COOR_9$ in which $R_9$ represents a $C_{1-4}$ alkyl group or a benzyl group, with a hydrazine of formula VI

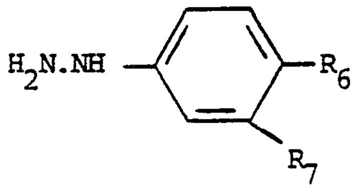

VI

4. A process according to claim 3 to prepare a compound wherein $R_5$ represents methyl, $R_6$ represents bromo or trifluoromethyl and $R_7$ and $R_8$ each represent hydrogen.

5. A process to prepare compounds of formula I as claimed in claim 1 wherein $R_1$ and $R_2$ represent a bond, $R_3$ represents methyl, $R_4$ and $R_5$ represent hydrogen, $R_6$ represents hydrogen, halo, trifluoromethyl, a $C_{1-6}$ alkylthio group, a $C_{1-6}$ alkyl group or a $C_{1-6}$ alkoxy group; R7 represent hydrogen, halo or trifluoromethyl; and $R_8$ represents hydrogen, halo, trifluoromethyl, hydroxy or a $C_{1-6}$ alkyl group comprising reacting compounds of formula V

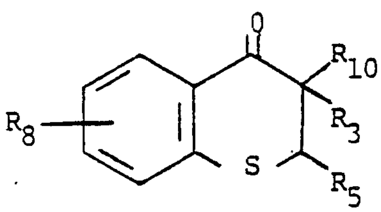

V

wherein $R_5$ represents hydrogen, $R_3$ represents methyl and $R_{10}$ represents carbamoyl or $COOR_9$, in which $R_9$ represents a $C_{1-4}$ alkyl group or a benzyl group, with a hydrazine of formula VI

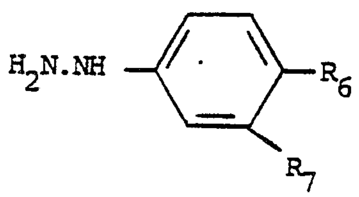

VI

6. A process according to claim 5 to prepare a compound wherein $R_6$ represents chloro, bromo or trifluoromethyl, $R_7$ represents hydrogen or chloro and $R_8$ represents hydrogen.

European Patent Office

# PARTIAL EUROPEAN SEARCH REPORT

which under Rule 45 of the European Patent Convention shall be considered, for the purposes of subsequent proceedings, as the European search report

Application number: EP 89307726.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | US - A - 4 268 516 (LOMBARDINO et al.) * Claims 1,11 * -- | 1,11 | C 07 D 495/04<br>A 61 K 31/415 |

TECHNICAL FIELDS SEARCHED (Int. Cl.4)

C 07 D 495/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-12, 14-19

Claims searched incompletely: -

Claims not searched: 13

Reason for the limitation of the search:

Method for treatment of the human or animal body by therapy; Art. 52(4) EPC

| Place of search | Date of completion of the search | Examiner |
|---|---|---|
| VIENNA | 10-10-1989 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document